Europäisches Patentamt

**European Patent Office**

**Office européen des brevets**

(11) Numéro de publication: **0 413 872 A1**

# (12) DEMANDE DE BREVET EUROPEEN

(21) Numéro de dépôt: **89440086.0**

(51) Int. Cl.5: **A61M 5/32**

(22) Date de dépôt: **23.08.89**

(43) Date de publication de la demande:
**27.02.91 Bulletin 91/09**

(84) Etats contractants désignés:
**AT BE CH DE ES FR GB GR IT LI LU NL SE**

(71) Demandeur: **Boisson-Müller, Jack**
**Domaine des Planes**
**F-83520 Roquebrune sur Argens(FR)**

(72) Inventeur: **Boisson-Müller, Jack**
**Domaine des Planes**
**F-83520 Roquebrune sur Argens(FR)**

(74) Mandataire: **Bossard, Jacques-René**
**Cabinet MEYER & COURTASSOL Bureau**
**EUROPE 20 Place des Halles**
**F-67000 Strasbourg(FR)**

(54) **Etui de protection destiné à l'usage des aiguilles et des seringues.**

(57) Dispositif de protection destiné à des seringues ou tout autre appareil muni d'une aiguille et assurant l'enveloppement total de l'aiguille, caractérisé en ce qu'il se compose :
- d'un embout supérieur (1) comprenant un moyen d'assurer le passage de l'aiguille à travers lui et un moyen d'imprimer un mouvement complexe à l'ensemble du dispositif de protection,
- d'un fourreau (2) doté de moyens permettant de revenir à la position initiale après déformation, ainsi que des moyens d'assurer le guidage et/ou le positionnement de l'aiguille,
et en ce que le fourreau est hermétiquement solidarisé à l'embout supérieur, d'une part, et à la base de fixation de l'aiguille, directement sur la base (6) de la seringue d'autre part.

Fig. 1

EP 0 413 872 A1

## ETUI DE PROTECTION DESTINE A L'USAGE DES AIGUILLES ET DES SERINGUES.

La présente invention a trait à un dispositif de protection destiné à des seringues ou à tout autre appareil muni d'une aiguille, assurant un enveloppement total de ladite aiguille de manière à prévenir les risques de contamination et/ou de blessures au contact des aiguilles.

Selon un second objectif, l'invention est conçue pour garantir aux praticiens (médecins, infirmiers, vétérinaires, etc...) les conditions d'hygiène et de sécurité indispensables à l'exercice de leur fonction. Bien entendu, celles-ci doivent corrélativement être garanties aux patients et plus généralement à toute personne susceptible d'être piquée accidentellement par une aiguille souillée.

L'actualité récente a montré à quel point le manque d'un tel dispositif se fait sentir, puisqu'actuellement aucune protection autre que le classique étui protecteur individuel de stérilité n'est prévue. Celui-ci, aisément enlevable, n'a pas été conçu dans l'esprit d'inciter les utilisateurs à le remettre après usage, quelle que soit alors la destination de la seringue. Sa seule fonction est en réalité de masquer le bout effilé de l'aiguille. Or, la manipulation de l'aiguille (pour la jeter) ou le repositionnement de l'étui protecteur après l'utilisation de l'aiguille représente des risques de blessures accidentelles d'autant plus graves que l'aiguille peut être souillée par du sang contaminé.

Il n'en reste pas moins que, au travers de phénomènes sociologiques comme la toxicomanie, il arrive fréquemment que des seringues décapuchonnées soient abandonnées dans des lieux fréquentés, tels que des plages publiques, des parcs, etc...

Ces seringues, souvent laissées par des populations à risque tels que des toxicomanes séropositifs, représentent un risque réel que l'invention se propose de combattre, ajoutant de surcroit à la protection mécanique de l'aiguille une meilleure garantie du maintien de la stérilité.

A cet effet, le dispositif de protection selon la présente invention se compose de deux éléments principaux :

- un embout supérieur comprenant un moyen d'assurer le passage de l'aiguille à travers lui et un moyen d'imprimer un mouvement complexe à l'ensemble du dispositif de protection,

- un fourreau doté de moyens permettant de revenir à la position initiale après déformation, ainsi que des moyens d'assurer le guidage et/ou le positionnement de l'aiguille.

Au surplus, le fourreau est hermétiquement solidarisé à l'embout supérieur, d'une part, et à la base de fixation de l'aiguille sur la seringue ou à la base de la seringue, d'autre part. Ces trois éléments peuvent être réalisés par moulage en une seule pièce, dont l'épaisseur peut varier, à savoir, par exemple :
- en latex plus épais, résistant à la pénétration pour l'embout
- en latex moins épais, assurant l'effet ressort pour le fourreau.

Selon une configuration possible, le moyen d'assurer le passage de l'aiguille dans l'embout supérieur consiste en un orifice de diamètre légèrement supérieur à celui de l'aiguille, prolongé le cas échéant par un conduit inférieur de guidage de l'aiguille. Ledit conduit peut prendre des formes variées selon l'utilisation et le degré de perfectionnement du dispositif.

Ainsi, il peut n'être qu'une simple tubulure prolongeant l'orifice, ou prendre une forme cônique ou sphérique, ou encore être positionné de biais dans la suite d'un orifice pratiqué également de biais dans l'embout supérieur.

Cet orifice est excentré par rapport à l'axe vertical de l'aiguille, de telle sorte que le mécanisme ne puisse pas se déclencher seul, c'est-à-dire que le dégagement de l'aiguille ne puisse se produire sans une intervention précise de l'utilisateur.

Selon une variante possible du dispositif de protection selon l'invention, l'orifice en question peut être obturé par un procédé tel que le trempage, le bouchonnage ou encore par application d'un film. La conséquence en est une protection par fermeture hermétique assurant le maintien de la stérilisation compatible avec l'utilisation future du dispositif. Bien entendu, cette obturation doit être prévue pour ne pas résister à une pression raisonnable de l'aiguille, obtenue en manipulant l'ensemble du dispositif pour amener l'aiguille en face de l'orifice obturé, par un mouvement complexe que l'on peut schématiquement décomposer en une rotation et deux translations selon deux directions orthogonales, horizontale et verticale. Par ailleurs, il est évident que le matériau choisi pour réaliser l'embout supérieur doit résister aux pressions intempestives de l'aiguille. On peut bien entendu utiliser le même matériau que pour le fourreau, mais plus épais pour assurer la non pénétration.

Il est également possible, afin d'augmenter la sécurité d'emploi, de prévoir une languette amovible sous l'orifice, écarté au cours du mouvement relatif entre la seringue et le dispositif de protection. La languette joue alors en plus un rôle dans le retour à la position initiale, c'est-à-dire le mouvement relatif inverse.

Les mouvements complexes dont on a fait mention sont rendus possibles par les caractéristiques mécaniques du fourreau. Celui-ci doit posséder des qualités d'élasticité lui conférant notamment un "effet" ressort permettant, ainsi qu'on l'a vu, le retour à la position relative initiale, aiguille totalement protégée, sans contact possible avec un utilisateur. La compression, la torsion et plus généralement les déformations que l'on veut imprimer au dispositif de protection sont exercées par le biais d'un ergot placé sur le côté de l'embout supérieur, ou par action directe sur ledit embout.

Ces différents effets de déformation sont assurés par des ressorts, soit en plastique type "accordéon", soit en matériau métallique à boudin comme par exemple les ressorts de compression classiques. Ces ressorts constituent l'armature du fourreau, dont l'habillage est constitué par une gaine souple, par exemple en plastique, capable d'encaisser également les efforts (autant de torsion que de translation) avec une durée de vie compatible avec celle du produit. Bien entendu, selon les caractéristiques initialement posées, ces matériaux doivent permettre un retour à la forme initiale sans intervention particulière de l'utilisateur, dès lors que cessent les forces exercées.

Le fourreau peut lui-même être le ressort, s'il est en latex, P.E., etc...

Additionnellement, le fourreau peut comporter des dispositifs de guidage et de positionnement de l'aiguille, de telle sorte que le mouvement relatif seringue/dispositif de protection soit facilité, dans un sens comme dans l'autre. Il peut également s'agir d'un positionnement simple, destiné à accroître la sécurité d'utilisation, en maintenant la pointe de l'aiguille à une position diamétralement opposée à celle de l'orifice.

En ce qui concerne la fabrication de ces dispositifs, il est à noter que la solidarisation du fourreau aux deux extrémités peut se faire soit directement, soit par l'intermédiaire d'une bague augmentant l'étanchéité, et par collage, soudure, vissage, etc..., soit encore par moulage en une seule pièce.

Quant à la liaison supérieure, elle peut également être réalisée d'une seule pièce, par exemple par moulage.

On va maintenant procéder à une description plus détaillée du dispositif selon l'invention, en se référant aux dessins présentés en annexe, pour lesquels :

- la figure 1 présente une vue perspective schématique de l'ensemble du dispositif,
- les figures 2a et 2b décomposent le mouvement complexe permettant l'extraction de l'aiguille,
- la figure 3 représente un embout supérieur muni d'une prolongation simple,
- les figures 4, 5 et 6 montrent trois autres types de prolongation inférieure,

- la figure 7 précise l'utilisation d'une languette de protection,
- les figures 8a et 8b représentent l'utilisation d'une bague de guidage et positionnement et,
- la figure 9 montre un exemple de tube intérieur de positionnement de l'aiguille.

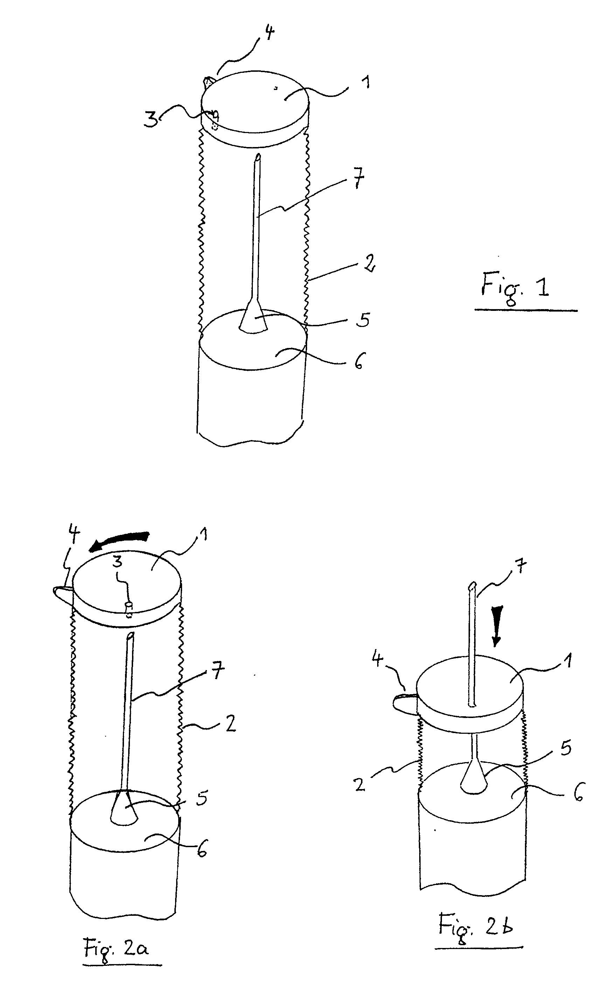

Selon la configuration schématique montrée en figure 1, le dispositif de protection se compose de l'embout supérieur (1) et du fourreau (2). L'orifice (3) pratiqué dans l'embout est largement excentré, et se situe à un emplacement proche de la périphérie. Un ergot (4) est rapporté sur le côté de l'embout supérieur (1), permettant de communiquer le mouvement complexe de l'ensemble. Dans cette configuration, le fourreau (2) est fixé sur la base de la seringue (5) et par conséquent il n'y a pas de réduction du diamètre du fourreau, comme cela serait nécessaire si celui-ci venait se fixer sur la base de fixation de l'aiguille sur la seringue (5).

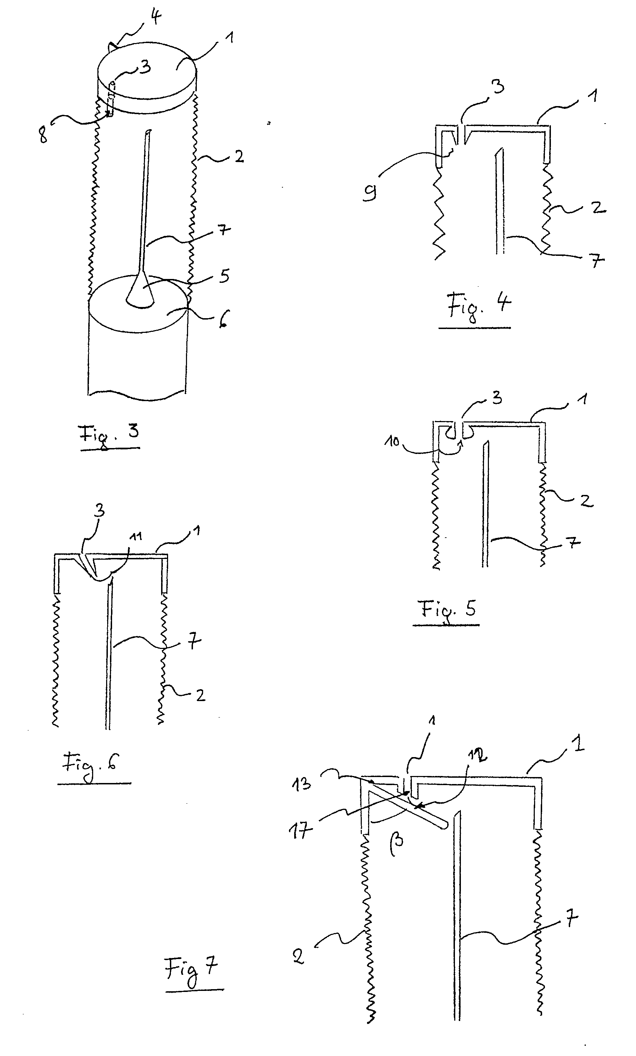

Comme cela apparait sur les figures 2a et 2b, le mouvement se décompose comme suit : il faut au préalable amener l'orifice (3) en vis à vis de l'aiguille (7) en imprimant au fourreau un mouvement résultant d'une torsion, suivi d'une flexion recentrant ledit orifice (3).

Lorsque ceci est réalisé, il n'y a plus qu'à effectuer une translation de haut en bas afin de permettre à l'aiguille de sortir. Il est possible de la dénuder sur la longueur voulue. Après toute utilisation, et sans intervention aucune de l'utilisateur, l'aiguille rentre automatiquement son logement et il ne subsiste aucun risque de se piquer accidentellement.

La figure 3 donne un exemple de prolongation inférieure (8) simple, alors que les figures suivantes (figures 4 à figure 6) montrent des variantes possibles. Bien évidemment, l'extrémité inférieure de ce tuyau prolongateur (8) est placée à une hauteur supérieure à celle de la pointe de l'aiguille, de sorte que les manipulations à effectuer pour sortir l'aiguille ne nécessitent pas une flexion trop intense du fourreau. En effet, dans le cas contraire, après utilisation l'aiguille ne ressortirait pas du tuyau mais y resterait et pourrait se dégager sous simple pression.

Les améliorations apportées à l'élément inférieur (8) ont pour but d'accroître encore la sécurité en empêchant la sortie intempestive de l'aiguille lors d'un choc ou de la chute de la seringue.

La portion de cône (9) ou la sphère (10) présentées respectivement en figures 4 et 5 peuvent bien entendu être combinées avec un conduit perforé de biais (11), tel que montré en figure 6. De la même manière, il est possible d'adjoindre une languette de protection (12) à l'une ou l'autre des configurations précédentes. .

Selon une variante de la figure 7, le tronçon de prolongation (8) est biseauté dans une direction parallèle à celle de la languette de protection (12).

Cette languette (12) a l'une de ses extrémités fixée à la cornière (13) de l'embout supérieur (1), l'autre étant libre. Elle fait un angle ß avec une génératrice du cylindre contenant le fourreau, qui lui confère une sorte d'"effet ressort". En outre, la languette maintien l'aiguille à distance de l'orifice de sortie, en position de repos.

Lorsqu'on actionne le dispositif, la pression latérale exercée par l'aiguille sur ladite languette plaque celle-ci contre une paroi du fourreau, laissant le champ libre à l'aiguille qui peut de la sorte se positionner dans l'axe de l'orifice ou, le cas échéant, du tube.

En fin d'action, la languette se détend et renvoie l'aiguille dans sa position initiale, dès lors qu'il n'y a plus de pression.

Selon une autre configuration, l'aiguille est située, en position de repos, sous ladite languette, et il faut alors exercer un mouvement combiné transversal et longitudinal pour la mettre en position de traverser l'orifice. Le dégagement de l'aiguille doit alors se faire suivant un axe vertical.

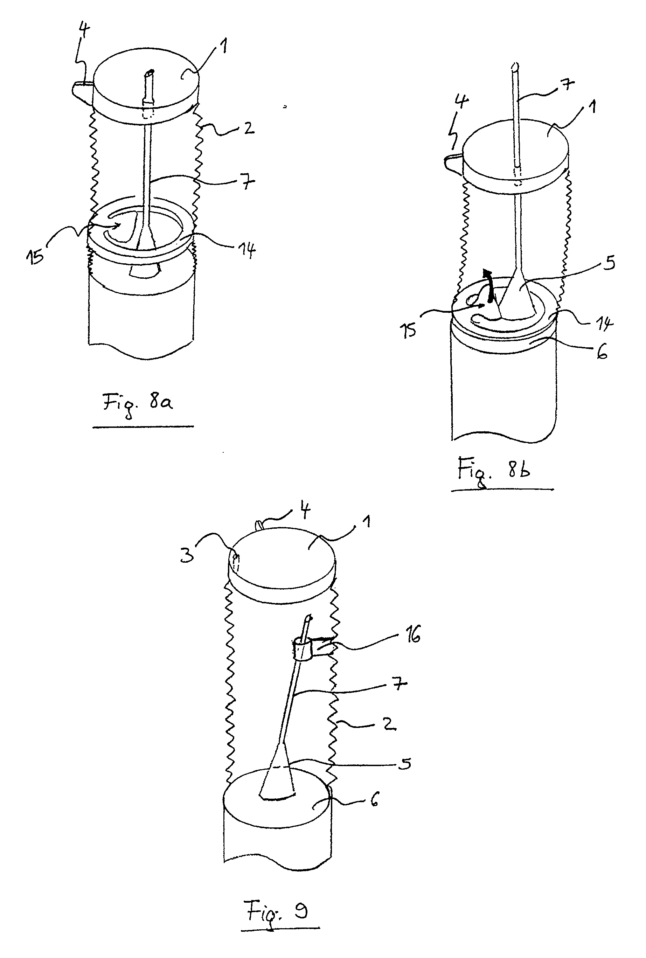

La figure 8 montre un anneau de positionnement (14) solidarisé au fourreau, situé en partie basse de celui-ci, et dont la fonction est de guider le mouvement relatif entre l'aiguille et l'ensemble du dispositif, de telle sorte que le mouvement complexe destiné à mettre l'aiguille dans l'axe de l'orifice s'en trouve simplifié. Selon la figure 8, il peut y avoir additionnellement un taquet (15) de rabat subissant une pression vers le haut de la part de la base de fixation de l'aiguille.

Cette languette (15) est alors rabattue vers la paroi du fourreau, permettant le dégagement maximal de l'aiguille, et aidant à retrouver la position initiale en fin d'action.

Enfin, la figure 9 montre un exemple de ce que pourrait être l'anneau ou tube intérieur (16), dans lequel s'insère l'aiguille, afin de maintenir un écart presque diamétral entre l'aiguille et l'orifice.

Bien entendu, l'invention ne se limite pas à cette description, et comporte également des variantes restant dans l'esprit qui a présidé à sa conception. Notamment, les combinaisons entre ces diverses caractéristiques doivent êtres considérées comme faisant partie intégrante de la présente invention.

## Revendications

1. Dispositif de protection destiné à des seringues ou tout autre appareil muni d'une aiguille et assurant l'enveloppement total de l'aiguille, caractérisé en ce qu'il se compose :
- d'un embout supérieur (1) comprenant un moyen d'assurer le passage de l'aiguille à travers lui et un moyen d'imprimer un mouvement complexe à l'ensemble du dispositif de protection,
- d'un fourreau (2) doté de moyens permettant de revenir à la position initiale après déformation, ainsi que des moyens d'assurer le guidage et/ou le positionnement de l'aiguille,
et en ce que le fourreau est hermétiquement solidarisé à l'embout supérieur, d'une part, et à la base de fixation de l'aiguille, directement sur la base (6) de la seringue d'autre part.

2. Dispositif de protection selon la revendication 1, caractérisé en ce que l'embout supérieur (1) comporte un orifice (3) de diamètre légèrement supérieur à celui de l'aiguille (7), décentré et à distance de l'aiguille, un ergot (4) étant au surplus rapporté sur le côté de l'embout (1) afin de permettre la communication d'un mouvement par l'intermédiaire du fourreau (2), ainsi que la mise en place de l'orifice en face de la pointe de l'aiguille puis le dégagement vertical de l'ensemble.

3. Dispositif de protection selon la revendication 1, caractérisé en ce que le fourreau (2) est un ressort en plastique type "accordéon", ou une simple gaine présentant les qualités élastiques requises au fonctionnement.

4. Dispositif de protection selon la revendication 1, caractérisé en ce que le fourreau (2) est un ressort à compression métallique type boudin recouvert d'une gaine en plastique souple et résistante.

5. Dispositif de protection selon la revendication 2, caractérisé en ce que l'orifice (3) de diamètre légèrement supérieur à celui de la seringue pratiqué dans l'embout (1) est prolongé vers le bas par un tuyau (8) de même diamètre interne dont l'extrémité inférieure est placé à hauteur supérieure à l'extrémité haute de l'aiguille en position de repos de l'ensemble.

6. Dispositif selon la revendication 5, caractérisé en ce que le tuyau de prolongation (8) vers le bas du trou (3) de l'embout (1) est de forme conique (9).

7. Dispositif selon la revendication 5, caractérisé en ce que le tuyau de prolongation (8) vers le bas du trou (3) de l'embout (1) prend la forme d'une sphère (10) alésée en son axe vertical.

8. Dispositif selon la revendication 5, caractérisé en ce que le conduit constitué par le trou (3) pratiqué dans l'embout (1) prolongé d'une tuyauterie (8) à un axe oblique (11).

9. Dispositif selon l'une des revendications 3 ou 4, caractérisé en ce que le fourreau (2) est muni à sa partie inférieure, au niveau du haut de la base de fixation (5) de l'aiguille, d'une bague de guidage (14) et de positionnement comportant le cas échéant une languette interne (15) pouvant être rabattue par pression de la base de fixation de l'aiguille.

10. Dispositif selon l'une des revendications 3 ou 4, caractérisé en ce que le fourreau (2) comporte un anneau ou un tube inférieur (16), de diamètre légèrement supérieur au diamètre de l'aiguille, de manière à positionner l'aiguille au repos et à la guider lors du déplacement relatif entre l'embout et l'aiguille.

11. Dispositif selon la revendication 2, caractérisé en ce que l'embout (1) est prolongé vers le bas par un conduit asymétrique biseauté (17), la paroi la plus centrale étant plus longue que l'autre, et en ce qu'il est muni d'une languette inclinée (12) prenant place sous le conduit biseauté et rabattable pour permettre le passage de l'aiguille, par simple pression de celle-ci.

12. Dispositif selon l'une quelconque des revendications précédentes, caractérisé en ce que les parois latérales de l'embout (1) ont une dimension telle que la pointe de l'aiguille ne puisse transpercer le fourreau (2) en cas d'oscillation de l'aiguille.

Fig. 1

Fig. 2a

Fig. 2b

Fig. 3

Fig. 4

Fig. 5

Fig. 6

Fig 7

Fig. 8a

Fig. 8b

Fig. 9

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | EP-A-0 321 903 (DOLGIN)<br>* Colonne 7, ligne 50 - colonne 8, ligne 56; figures 4-6 * | 1,3,15 | A 61 M 5/32 |
| A | | 2,5,6 | |
| X | DE-A-3 808 688 (HAGEN)<br>* Colonne 5, lignes 24-52; colonne 6, lignes 11-32; colonne 8, lignes 34-52; figuren * | 1,3,15 | |
| A | | 2,5,6, 11 | |
| X | US-A-4 795 432 (KARCZMER)<br>* Colonne 3, ligne 47 - colonne 4, ligne 21; figures 1-4 * | 1,3,4, 15 | |
| A | GB-A-2 202 747 (DUCAT)<br>* Page 9; figure 1 * | 1,2,4 | |
| A | WO-A-8 702 254 (PHYSIONIC GESELLSCHAFT FUR MEDIZIN- UND SYSTEMTECHNIK GmbH)<br>* Page 11, lignes 1-23; figures 1-3 * | 1,3,4 | **DOMAINES TECHNIQUES RECHERCHES (Int. Cl.5)**<br><br>A 61 M |

Le présent rapport a été établi pour toutes les revendications

| Lieu de la recherche | Date d'achèvement de la recherche | Examinateur |
|---|---|---|
| LA HAYE | 14-02-1990 | CLARKSON P.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P0402)